# EUROPÄISCHE PATENTSCHRIFT

(11) **EP 2 926 011 B1**
(45) Veröffentlichungstag und Bekanntmachung des Hinweises auf die Patenterteilung: **06.11.2019**
(21) Anmeldenummer: 13802550.7
(22) Anmeldetag: 19.11.2013
(51) Int. Cl.: F04D 23/00, A61M 16/00, F04D 29/18, F04D 29/26, F04D 29/66

(54) **KLEINER, GERÄUSCHARMER SEITENKANALVERDICHTER, INSBESONDERE FÜR GERÄTE IN DER BEATMUNGSTHERAPIE**
SMALL, LOW-NOISE SIDE CHANNEL COMPRESSOR, IN PARTICULAR FOR DEVICES IN VENTILATION THERAPY
COMPRESSEUR À CANAL LATÉRAL PLUS PETIT ET MOINS BRUYANT, EN PARTICULIER POUR DES APPAREILS EN THÉRAPIE RESPIRATOIRE

(30) Priorität: 29.11.2012 DE 102012023347
(43) Veröffentlichungstag der Anmeldung: 07.10.2015
(73) Patentinhaber: TNI medical AG, 97084 Würzburg (DE)
(72) Erfinder: EBERHARD, Dietmar, 79341 Kenzingen (DE); ANGER, Ewald, 97246 Eibelstadt (DE)
(74) Vertreter: advotec.
(86) Internationale Anmeldenummer: PCT/EP2013/074134
(87) Internationale Veröffentlichungsnummer: WO 2014/082892

(56) Entgegenhaltungen:
- EP-A1- 2 128 200
- EP-A2- 1 519 050
- WO-A1-02/16776
- WO-A1-2010/133866
- DE-A1- 3 823 514
- DE-A1- 19 649 529
- DE-A1-102006 024 839
- DE-U1- 9 207 883
- JP-A- 2002 339 889
- US-A1- 2003 118 438

## Beschreibung

Seitenkanalverdichter der vorliegenden Art bestehen in der Regel aus einem Gehäuse mit einem darin befindlichen ringförmigen, im Querschnitt halbreisförmigen Kanal, "Seitenkanal" genannt, zum Verdichten eines Gases. Zwischen einer Gas-Einlass-Öffnung (Saugseite) und einer Gas-Auslass-Öffnung (Druckseite) ist der Seitenkanal durch einen sogenannten Unterbrecher getrennt. Über dem Seitenkanal liegt ein elektromotorisch drehangetriebenes Laufrad. In Drehrichtung gesehen befinden sich im Laufrad mehrere radial angeordnete Schaufeln auf einem Teilkreisring, die den Teilkreisring in entsprechende Zellen, sogenannte Schaufelkammern, aufteilen.

Durch die Gas-Einlass-Öffnung tritt ein Gas, insbesondere Luft, in den Seitenkanal ein, wobei ein Teil der Gasmoleküle durch eine Schaufel in tangentialer Richtung mitgerissen wird. Aufgrund der durch eine hohe Laufraddrehzahl verursachten, auf die betrachteten Gasmoleküle einwirkenden Fliehkraft werden diese radial nach außen beschleunigt und fließen derart beschleunigt aus der Schaufel in den Seitenkanal hinein. Hier werden sie in Richtung des Laufrades umgelenkt und erfahren durch die nächste Schaufel des Laufrades eine weitere Beschleunigung, so dass sich die Gasmoleküle auf einer torusförmig gebogenen Schraubenbahn von der Gas-Einlass-Öffnung zur Gas-Auslass-Öffnung bewegen, wobei der Druck im Gas stetig ansteigt. Der Unterbrecher minimiert die Gasmenge, die von der Gas-Auslass-Öffnung zur Gas-Einlass-Öffnung befördert wird.

Seitenkanalverdichter sind aufgrund Ihrer Wartungsfreiheit, des ölfreien Verdichtungsbetriebes und ihrer Langlebigkeit sowie der Option eines hohen Druckaufbaus hervorragend geeignet zur Verwendung in Geräten zur transnasalen Insufflation, sog. TNI-Geräten, wie sie beispielsweise aus der DE 10 2006 024 839 A1 oder der DE 101 05 383 C2 bekannt sind.

Neben den oben genannten Vorteilen von Seitenkanalverdichtern weisen diese aber im Vergleich zu Radialverdichtern schlechtere Wirkungsgrade auf, da durch die stark turbulente Strömung im Seitenkanalverdichter mechanische Energie des Laufrades zu einem hohen Anteil in drucktechnisch nicht nutzbare Wärme umgewandelt und durch die Bauform des Verdichters in den Laufradschaufeln komprimierte Luft von der Druck- auf die Saugseite mitgeschleppt wird, was ebenfalls zu einer Wirkungsgradminderung führt.

Das Hauptproblem der Seitenkanalverdichter besteht aber in extrem hohen Geräuschemissionen, die zum einen von der instationären Turbulenzströmung, die ein rauschartiges Spektrum über den gesamten Frequenzbereich aufweist, herrühren und zum anderen von darüber überlagerten seitenkanaltypischen tonalen Schallkomponenten stammen. Zu Letzteren gehören Vibrationen des Gehäuses und des Laufrades aufgrund von Unwuchten, die der Grundfrequenz und einem Vielfachen der Grundfrequenz der Rotation des Laufrades entsprechen, und der sogenannte "Schaufelton". Da dieser durch die Druckschwankungen am Unterbrecherein- und -austritt zustande kommt, die von jeder einzelnen Schaufel induziert werden, entspricht die Frequenz dieses Tones der Umdrehungsfrequenz des Laufrades multipliziert mit der Anzahl der Schaufeln. Analog zu den Harmonischen der Grundfrequenz besitzt der Schaufelton Seitenbänder im Abstand der Umdrehungsfrequenz. Als hochfrequenter Ton ist der Schaufelton besonders störend.

### Stand der Technik

Bisherige Entwicklungen von Seitenkanalverdichtern der vorliegenden Art richteten sich insbesondere auf die Beseitigung der oben genannten Mängel.

So ist in der DE 196 49 529 A1 ein Seitenkanalverdichter offenbart, bei dem zur Verbesserung des Wirkungsgrades im einlassseitigen Endabschnitt des Seitenkanals ein Leitelement ausgebildet ist, welches eine effektivere Mitnahme des zu fördernden Gases erlaubt. Aus der EP 1 703 136 B1 ist ein Seitenkanalverdichter mit komplexer Seitenkanalgeometrie mit Hinterschneidungen bekannt, bei dem ein Einlegeteil dazu dient, die Oberflächengüte der Wandbereiche des Seitenkanals zu verbessern, um eine möglichst turbulenzfreie Strömungsführung des Gases zu erzielen, um dadurch den Wirkungsgrad zu verbessern. Bei dem aus der DE 199 13 950 A1 bekannten Seitenkanalverdichter wird ein gesteigerter Wirkungsgrad dadurch erzielt, dass sich die maximale Kanaltiefe in Richtung der Auslassöffnung kontinuierlich verringert und wenigstens ein Abschnitt vorgesehen ist, in dem der Seitenkanal einen Querschnitt in Form einer Ellipsenhälfte aufweist, wodurch es zu einem höheren Volumenstrom und einer erhöhten Verdichterleistung kommt. Aus der DE 10 2006 041 557 A1 ist bekannt, die Schaufeln des Laufrades jeweils derart dreidimensional zu formen, dass sie eine weitgehend wirbelfreie, räumlich verwundene Schaufelströmungsführung bilden, die auf die zu erbringende Leistung angepasst ist. Weitere bekannte Seitenkanalverdichter beschäftigen sich zur Verbesserung des Wirkungsgrades mit der Verringerung des sog. axialen und radialen Spaltmaßes. Durch die rotationsbedingten Spalten zwischen Gehäuse und Schaufelrad fließt Gas von der Druckseite auf die Saugseite und vermindert dadurch den Wirkungsgrad. Während aus der DE 20 2004 019 071 U1 bekannt ist, hierzu im Gehäuseteil oder im Laufrad eine umlaufende und zum Dichtspalt orientierte Nut vorzusehen, in der ein in den Dichtspalt reichender Dichtring aus einem elastischen Material formschlüssig eingelegt ist, schlägt die DE 10 2005 040 305 A1 vor, mindestens eine der einander gegenüberliegenden Flächen von Laufrad und Arbeitskammer des Gehäuses als raue Fläche auszubilden, deren Rauheit im Bereich von vorzugsweise 53 bis 45 µm liegt.

Besondere Maßnahmen zur Geräuschemissionsminderung sind aus der EP 2 207 967 B1, der WO 2006/039894 A2 und der US 3,951,567 bekannt. So lehrt die EP 2 207 967 B1, zur Geräuschemissionsreduzierung in mindestens einer Laufradschaufel in ihrem freien Randbereich mindestens eine Strömungs-Ausnehmung als Strömungs-Nut mit einem im Wesentlichen rechteckigen Querschnitt vorzusehen und dass nur 30 % bis 70 % aller Schaufeln des Laufrades eine Nut aufweisen sollen, um Gas-Turbulenz-Strukturen zu vermindern. Die WO 2006/039894 A2 zeigt neben Maßnahmen zur Spaltmaßverringerung durch ein Tellerfeder-Mutter-System oder eine Taumeleinrichtung ein Laufrad für einen Seitenkanalverdichter der vorliegenden Art auf, wobei in den Schaufelkammern kleinere Zwischenschaufeln zur Lärmreduzierung vorgesehen sind. Die US 3,951,567 offenbart einen Seitenkanalverdichter, der eine uneinheitliche Verteilung der Schaufeln auf dem Schaufelrad zur Geräuschemissionsreduzierung vorsieht. Aus der JP 2002 339889 ist eine Pumpe bekannt, die ein Schaufelrad aufweist, das eine Vielzahl von Schaufelkammern trägt, die durch Schaufelkammerwände getrennt sind. Diese Schaufelkammerwände können nach radial außen dicker werden, wodurch eine bessere Entfernbarkeit beim Spritzgießen erreicht werden soll.

Bei Geräten zur Flow Therapie (z. B. bekannt unter den Begriffen transnasale Insufflation TNI, Highflow Therapie HFT, Nasal Highflow NHF, transnasal Highflow, THF) werden definierte konstante Volumenströme (Gasströme) benötigt, die die bisher bekannten, oben aufgeführten geräuscharmen Seitenkanalverdichter aufgrund ihrer Schaufelkammer-Bauweise nur unzureichend erbringen. Gleichzeitig muss bei der Generierung des Volumenstromes insbesondere auf Geräuschemissionsverminderung geachtet werden, da z.B. durch ein Antischnarchgerät (DE 101 05 383 C2) der Partner des Schlafenden nicht durch Verdichtergeräusche anstelle von Schnarchgeräuschen gestört werden soll.

### Aufgabe und Lösung

Es ist daher Aufgabe der vorliegenden Erfindung, einen kleinen geräuscharmen Seitenkanalverdichter zur Erzeugung eines definierten, konstanten Volumenstromes (Luft/Gasstromes) für Geräte zur Beatmungstherapie zu schaffen.

Diese Aufgabe wird mit dem im Patentanspruch 1 angegebenen Seitenkanalverdichter gelöst. Vorteilhafte Ausgestaltungen der Erfindung sind Gegenstand der Unteransprüche 2 bis 11.

Der Seitenkanalverdichter nach dem vorliegenden Patentanspruch 1 zeichnet sich insbesondere durch die Form seiner Schaufelkammern und die sie trennenden, zum Laufradumfang hin dicker werdenden Schaufelkammerwände (Schaufeln) aus, wobei sich deren ins Innere benachbarter Schaufelkammern gerichtete Oberflächen parallel zu den radial verlaufenden Mittelebenen dieser Schaufelkammern erstrecken. Neben dem Vorteil, dass jede der Schaufelkammern jeweils in einem Arbeitsgang komplett durch z. B. einen Kugelfräser hergestellt werden kann, tragen sie insbesondere zu einem konstanteren Gasfluss und zur Geräuschverminderung bei, wie nachfolgend erläutert.

Im Stand der Technik weisen die Schaufeln des Laufrades eine konstante Wandstärke auf (s. z.B. DE 196 49 529 A1). Folglich nimmt die Breite der Schaufelkammern mit zunehmendem radialem Abstand zur Drehachse des Laufrades zu. Jede Schaufelkammer wird in Umfangsrichtung des Laufrades nach außen breiter. Auch sind die Bodenflächen der Schaufelkammern in Umfangsrichtung des Laufrades eben. Das einströmende Gas fließt an der einen Schaufel zum Schaufelkammerboden, dann, um 90° umgelenkt, den Schaufelkammerboden entlang, wird wieder um 90° umgelenkt, um an der nächsten Schaufel vom Schaufelkammerboden weg auszutreten. Diese Strömung wird durch eine radiale Strömung von der radial inneren Seite der Schaufelkammer zur radial äußeren Seite der Schaufelkammer überlagert. Diese radiale Strömung erfolgt durch die Fliehkräfte, welche auf die Gasmoleküle wirken und führen zu einer Druckerhöhung an der radialen Außenwand der Schaufelkammer. Ausgehend von einem kompressiblen Gas bewirkt die Verbreiterung der Schaufelkammer in Umfangsrichtung eine Gasdruckreduzierung, da sich das Gas entspannt, so dass eine aufgrund der Fliehkraft mögliche Druckerhöhung nicht vollständig erreicht wird, wenn von einer gleichen Anzahl von eintretenden und austretenden Molekülen, sprich Masseteilchen, ausgegangen wird.

Die Erfindung vermeidet diese zwei vorstehend dargestellten Effekte erheblich dadurch, dass die Breite der Schaufelkammern durch die vorgesehenen Maßnahmen in Umfangsrichtung konstant bleibt und folglich die Entspannung des Gases in radialer Richtung nicht vorhanden ist. Aufgrund der Fertigung der Schaufelkammern mit z. B. einem Kugelfräser liegt kein ebener Kammerboden vor. Der halbkreisförmige Schaufelkammerboden führt die an der einen Schaufel einströmende Luft zur gegenüberliegenden Schaufel, an der das Gas austritt, kontinuierlicher und folglich strömungstechnisch besser. Insgesamt wird dadurch eine erheblich weniger gestörte Gesamtströmung erzielt, welche im Ergebnis zu einem konstanteren Fluss der Gasmenge bei geringerer Geräuschentwicklung führt. Zwar verringert sich aufgrund der vorgeschlagenen Schaufelkammergeometrie der Wirkungsgrad im direkten Vergleich zu den aus dem Stand der Technik bekannten Geometrien, da für die Zirkulationsströmung weniger Raum zur Verfügung steht; dieser Nachteil wird aber durch weitere technische Ausgestaltungen aufgefangen. Um den Bauraum eines Gerätes, in das der erfindungsgemäße Seitenkanalverdichter zu integrieren ist, klein zu halten, ist ein Durchmesser des Laufrades von ca. 50 mm vorgesehen.

Die weiteren, in den Unteransprüchen 2 und 6 angegebenen Merkmale, dass die Schaufelkammern in radialer Richtung jeweils einen im Querschnitt halbkreisförmigen Boden aufweisen, der dem halbkreisförmigen Querschnitt des Seitenkanals entspricht, und die Übergänge zwischen den Böden der einzelnen Schaufelkammern und deren Schaufelkammerwänden mit Kurven versehen sind und dass die Oberflächen der gasführenden Teile, insbesondere der Seitenkanal und die Schaufelkammern, hydraulisch glatt ausgeführt sind, dienen zur Erhöhung des Wirkungsgrades aufgrund der bereits erwähnten besseren Strömungseigenschaften. Dabei ist anzumerken, dass z. B. die Verwendung eines Kugelfräsers bei der Herstellung eines Laufrades sehr vorteilhaft gegenüber der sonst angewandten üblichen Gießtechnik ist, da sich direkt glatte Oberflächen ergeben, die keinerlei Nachbehandlung bedürfen. Bei Einsatz z. B. eines Kugelfräsers erhalten die beanspruchten Kurven die Form eines Radius.

Durch die hohe Drehzahl n des Laufrades von circa 30000 1/min (Unteranspruch 5), die gleichzeitig den meisten Lärm verursacht, und mindestens 25, vorzugsweise 36, auf dem Laufrad befindlichen Schaufelkammern (Unteranspruch 4) ergibt sich ein Schaufelton, der bei 36 Schaufelkammern beispielsweise bei 18 kHz liegt, also außerhalb des menschlichen Hörbereichs. Gleichzeitig mit der Lärmverschiebung in den nicht hörbaren Bereich reduziert sich durch die quadratische Abhängigkeit des Druckes von der Drehzahl auch der erforderliche Durchmesser des Laufrades und wird auf einen Durchmesser von circa 50 mm festgelegt. Durch diese Maßnahme kann z. B. der Bauraum eines TNI-Gerätes, in das ein Seitenkanalverdichter zu integrieren ist, klein gehalten werden, was einen entscheidenden Vorteil mit sich bringt. Unterstützt wird dieser Vorteil dadurch, dass das Laufrad und das Gehäuse mindestens einen Teilkreis (Unteranspruch 3) aufweisen.

Um unerwünschte gesundheitliche Nebenwirkungen auszuschließen, sind die Oberflächen der gasführenden Teile, insbesondere der Seitenkanal und die Schaufelkammern, antibakteriell ausgeführt.

Aufgrund der im Unteranspruch 8 genannten glatten Oberflächen des Gehäuses und des darauf angeordneten Laufrades verringert sich das Spaltmaß. Aufgrund der ebenfalls in Unteranspruch 8 angegebenen Weiterbildung, dass am Ende des Unterbrechers eine Expansionsstrecke des verdichteten Gases vorgesehen ist, ist eine sanfte Entspannung des Gases gegeben, damit - ähnlich dem bekannten Prinzip des Diffusors - Gasgeschwindigkeit verlustarm in Druck umgesetzt werden kann. Beide Maßnahmen dienen somit ebenfalls zur Erhöhung des Wirkungsgrades.

Durch die im Unteranspruch 9 beanspruchte Ausgestaltung des Seitenkanalverdichters, dass an der Gas-Einlass-Öffnung und/oder der Gas-Auslass-Öffnung Schalldämpfer angeschlossen werden können, ergibt sich die Möglichkeit, im Bedarfsfalle zur Vermeidung übriger Schallemissionen bekannte, nach dem Prinzip eines Helmholtz-Resonators arbeitende Schalldämpfer oder Rohrschalldämpfer zusätzlich anzuschließen.

Die Verwendung von Werkstoffen geringer Dichte und gleichzeitig hoher Festigkeit, wie sie im Anspruch 10 beansprucht wird, dient beim Laufrad zur Erzielung geringer Fliehkräfte bei hohen Drehzahlen und zur Vermeidung störender Geräusche bei eventuellen Unwuchten. Zu diesen Werkstoffen zählen z. B. Aluminium, Magnesium und Peek.

Der Einbau des erfinderischen Seitenkanalverdichters in Geräten zur Beatmungstherapie wird ebenfalls beansprucht (Anspruch 11).

### Ausführungsbeispiel

Im Folgenden wird eine bevorzugte Ausführungsform der Erfindung unter Bezugnahme auf die beiliegenden Zeichnungen näher erläutert. Dabei zeigen:
**Fig. 1** Seitenkanalverdichter in Gesamtdarstellung
**Fig. 2** Seitenkanalverdichter im Schnitt
**Fig. 3** Anschlussseite des Seitenkanalverdichters
**Fig. 4** Schnitt A - A durch Gas-Einlass- und Gas-Auslass-Öffnungen
**Fig. 5** Gehäuseansicht deckelseitig
**Fig. 6** Vollschnitt des Gehäuses
**Fig. 7** Vollschnitt des Laufrades
**Fig. 8** Ansicht des Laufrad
**Fig. 9** Schnitt B - B durch das Laufrad

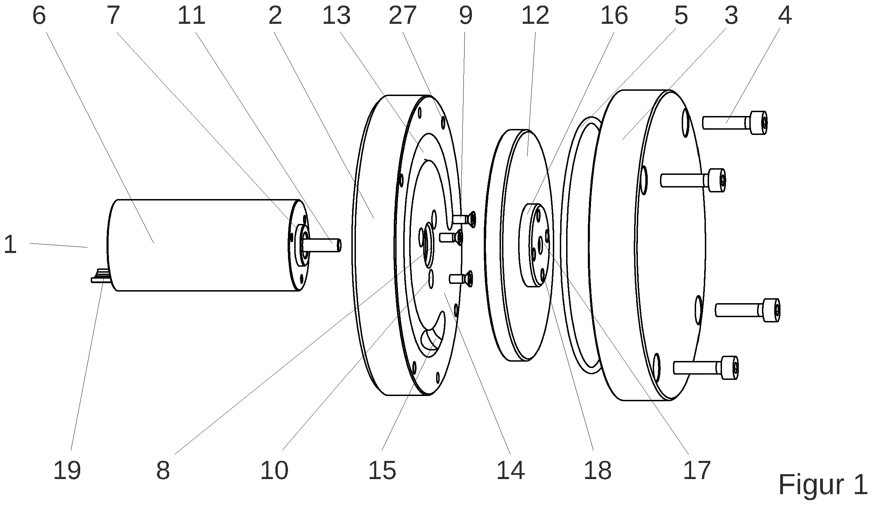

Figur 1 stellt den Seitenkanalverdichter (1) in Explosionsdarstellung dar. Mit einem Gehäuse (2) wird ein Gehäusedeckel (3) mittels Zylinderkopfschrauben (4) verschraubt. Ein Rundschnurring (5) bzw. O-Ring dichtet den Gehäusedeckel (3) gegen das Gehäuse (2) ab. Ein Elektromotor (6) ist mit seinem Zentrieransatz (7) in einer Zentrierbohrung (8) des Gehäuses (2) mit Senkkopfschrauben (9), welche Senkbohrungen (10) durchgreifen, befestigt und dreht mittels seines Motorzapfens (11) das darauf befestigte Laufrad (12). Im Gehäuse (2) ist ein Seitenkanal (13) mit dem diesen begrenzenden Unterbrecher (14) und der Expansionsstrecke (15) sichtbar. Das Laufrad (12) besitzt einen Zentrieransatz (16) sowie eine Bohrung (17) und Gewindebohrungen (18). Mit 19 ist der elektrische Anschluss für den Elektromotor (6) bezeichnet.

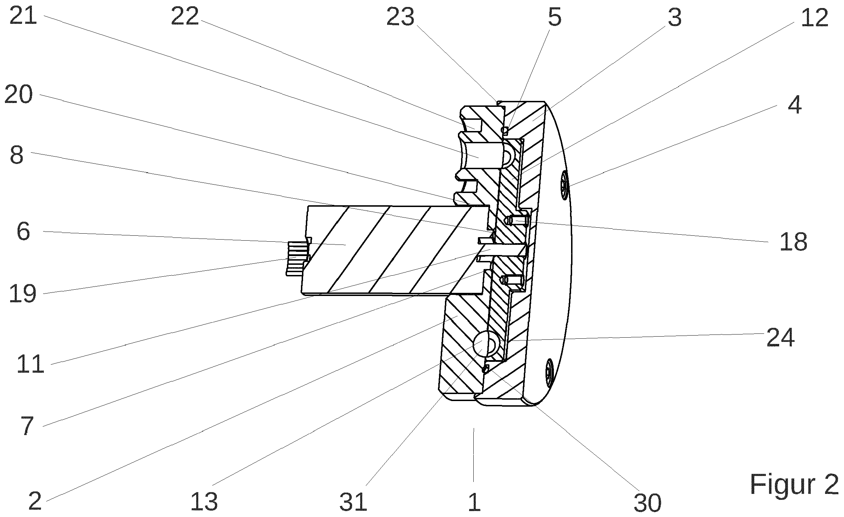

Figur 2 zeigt im Schnitt den Seitenkanalverdichter (1) im montierten Zustand. Im Gehäuse (2) mit Senkung (20) und der Zentrierbohrung (8) sitzt der Elektromotor (6) mit seinem Zentrieransatz (7). Am gegenüberliegenden Ende des Elektromotors (6) befindet sich der elektrische Anschluss (19) zu seinem Betrieb. Eine Gas-Einlass-Öffnung (21) mit diese umgebender Ringnut (22) zeigt der Schnitt ebenfalls. Der im Querschnitt (31) halbkreisförmige Seitenkanal (13) ist im Gehäuse (2) eingearbeitet. Der Gehäusedeckel (3) ist mittels Eindrehung (23) auf dem Gehäuse (2) zentriert und mit Zylinderkopfschrauben (4) verschraubt. Dargestellt sind noch die Gewindebohrungen (18). Abgedichtet wird der Gehäusedeckel (3) gegen das Gehäuse (2) durch den Rundschnurring (5), welcher in eine Ringnut (30) eingelegt ist. Auch sind die in radialer Ebene geschnittenen Schaufelkammern (24) im Laufrad (12) gezeigt, wobei diese dem Seitenkanal (13) und der Gas-Einlass-Öffnung (21) gegenüberliegen. Auf dem Motorzapfen (11) ist das Laufrad (12) befestigt.

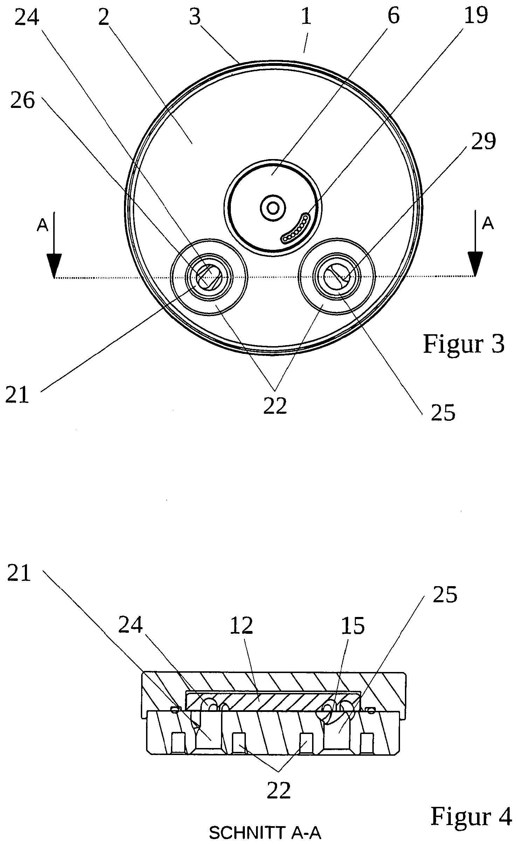

Figur 3 zeigt die Anschlussseite des Seitenkanalverdichters (1) mit dem Elektromotor (6), dem Anschluss (19), dem Gehäusedeckel (3), dem Gehäuse (2) und einer Gas-Einlass-Öffnung (21) und einer Gas-Auslass-Öffnung (25), mit den diese umgebenden Ringnuten (22). Eingezeichnet in der Gas-Einlass-Öffnung (21) sind noch eine Schaufelkammer (24) und eine Schaufel (26) sowie in der Gas-Auslass-Öffnung (25) eine Schaufelkammerwand (29) mit zum Laufradumfang hin dicker werdender Breite.

Figur 4 zeigt einen Schnitt A-A durch Gas-Einlass- und Gas-Auslass-Öffnungen (21, 25) des Seitenkanalverdichters (1). In diesen beginnt bzw. endet der Seitenkanal (13) mit der Expansionsstrecke (15). Gas-Einlass- und Gas-Auslass-Öffnungen (21, 25) münden ihrerseits in die Schaufelkammern (24) im Laufrad (12).

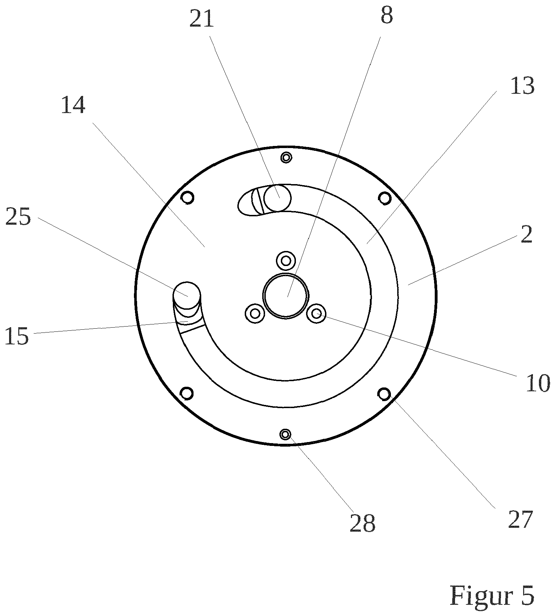

Figur 5 zeigt die Ansicht auf das Gehäuse (2) von der Seite, auf welche der Gehäusedeckel (3) aufgeschraubt wird. Der Seitenkanal (13) beginnt an der Gas-Einlass-Öffnung (21) und endet an der Gas-Auslass-Öffnung (25), wobei der Seitenkanal (13) einen Zentriwinkel von annähernd 270° hat. Der Unterbrecher (14) befindet sich zwischen den Enden des Seitenkanals (13). Weiterhin ist die Zentrierbohrung (8) zur Aufnahme des Zentrieransatzes (7) für den Elektromotor (6) gezeigt. Mit (10) sind die Senkbohrungen zum Verschrauben des Elektromotors (6) bezeichnet. Vier Gewindebohrungen (27) dienen zum Verschrauben des Gehäusedeckels (3). Die Gewindebohrungen (28) ermöglichen das Befestigen des Seitenkanalverdichters (1) insgesamt mittels Schrauben. Die Expansionsstrecke (15) bildet den Übergang vom Seitenkanal (13) zur Gas-Auslass-Öffnung (25).

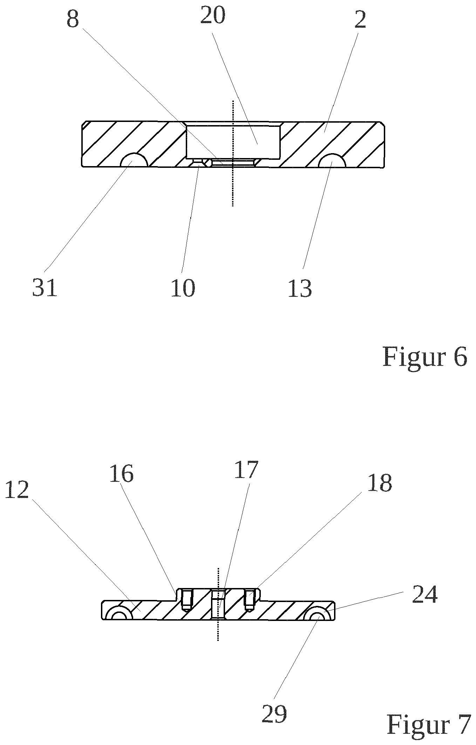

Figur 6 stellt das Gehäuse (2) im Vollschnitt dar. An die Senkung (20) zur Aufnahme des Elektromotors (6) schließt sich in axialer Richtung die Zentrierbohrung (8), welche vom Motorzapfen (11) durchgriffen wird, an. Die Senkbohrung (10) nimmt Senkschrauben (9) zum Befestigen des Elektromotors (6) auf. Ebenfalls ist der halbkreisförmige Querschnitt (31) des Seitenkanals (13) dargestellt.

Figur 7 zeigt das Laufrad (12) im Vollschnitt mit der Bohrung (17) zur Aufnahme des Motorzapfens (11) des Elektromotors (6). Die halbkreisförmige Schaufelkammer (24) ist in radialer Schnittebene unverzerrt dargestellt. Auch ist eine plane Schaufelkammerwand (29) mittels des kleineren Halbkreises angedeutet. Der gezeigte halbkreisförmige Ring, begrenzt innen von der Schaufelkammerwand (29) und außen von der Kontur der Schaufelkammer (24), hat eine Breite, die gleich dem Radius des Kugelfräsers ist, mit welchem die Schaufelkammer (24) gefräst wird. Auf den Zentrieransatz (16) und die Gewindebohrungen (18) sei noch hingewiesen.

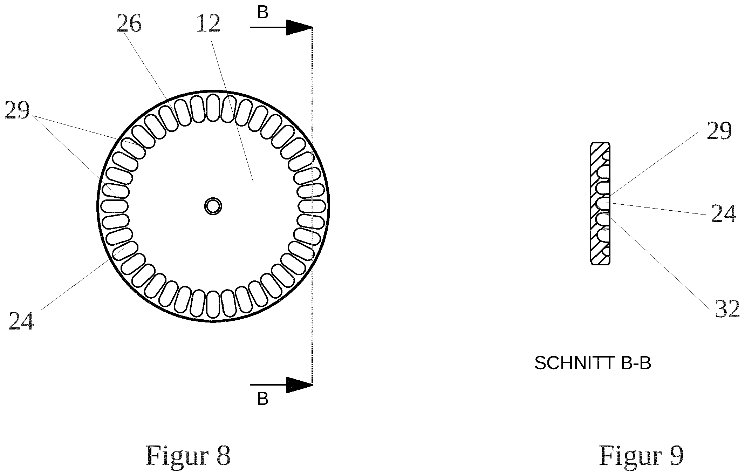

Eine axiale Ansicht des Laufrades (12) mit den Schaufelkammern (24) und den Schaufeln (26) ist in Figur 8 dargestellt. Im Ausführungsbeispiel sind 40 Schaufelkammern (24) vorhanden, deren radiale Erstreckung gleich ist. Die Form und Größe des Kugelfräsers bestimmt die konstante Breite der Schaufelkammer (24), wodurch plane Schaufelkammerwände (29) erzeugt werden, die sich parallel zur radial verlaufenden Mittelebene der Schaufelkammern (24) erstrecken. Aufgrund der konstanten Breite der Schaufelkammern (24) verbreitern sich die Schaufeln (26) in radialer Richtung nach außen. Diese Ausgestaltung führt dazu, dass jede Schaufelkammer (24) das gleiche Kammervolumen besitzt.

Den halbkreisförmigen Boden (32) der Schaufelkammer (24) zeigt der Schnitt B-B durch das Laufrad (12) in Figur 9. Die Kurve, hier der Radius dieses Bodens, wird vom Radius des Kugelfräsers bestimmt. Ebenfalls ist die plane Schaufelkammerwand (29) erkennbar. Alle Schaufelkammern (24) dringen gleich tief in das Laufrad (12) ein. Alternativ zur Herstellung mit einem Kugelfräser kann auch ein Erodierverfahren angewendet werden.

Für einige Anwendungen hat sich ein Laufraddurchmesser von 50 mm als besonders vorteilhaft erwiesen. Als Werkstoffe kommen diejenigen mit geringer Dichte und gleichzeitig hoher Festigkeit in Betracht, um geringe Fliehkräfte bei hohen Drehzahlen zu erzielen. Die mit dem Gas in Kontakt kommenden Flächen sind hydraulisch glatt und antibakteriell ausgeführt.

Im Betrieb dreht der Elektromotor (6) das Laufrad (12), wodurch Gas über die Gas-Einlass-Öffnung (21) in den Seitenkanal (13) angesaugt wird. Dies bewirken die Schaufelkammern (24), in welche die Gasmoleküle von der Gas-Einlass-Öffnung (21) kommend eintreten. Die Gasmoleküle werden, wie bei jeder Kreisbewegung, radial und tangential beschleunigt. Dies bewirkt ein Überlagern mehrerer Strömungskomponenten. Einerseits werden die Gasmoleküle radial nach außen aufgrund der auftretenden Zentrifugalkraft beschleunigt und erhöhen den Druck im äußeren Bereich der Schaufelkammer (24). Das komprimierte Gas tritt in den Seitenkanal (13) ein und wird der Wandung folgend auf einer Kreisbahn umgelenkt, um danach wieder in eine nachfolgende Schaufelkammer (24) einzutreten. Andererseits strömen die Gasmoleküle entlang der einen Schaufel (26) in axialer Richtung bezogen auf die Drehachse des Elektromotors (6), werden von dem halbkreisförmigen Boden (32) der Schaufelkammer (24) umgelenkt, um danach von der gegenüberliegenden Schaufel (26) in entgegengesetzter Richtung in den Seitenkanal (13) gepresst zu werden. Auch dies führt zu einer Druckerhöhung. Im Ergebnis wird eine wendelförmige Strömung erzeugt, wobei der Gasdruck stetig zunimmt, bis das Gas an der Gas-Auslass-Öffnung (25) austritt. Dazu dient auch der Unterbrecher (14), welcher die Gasströmung in Umfangsrichtung nahezu vollständig verhindert. Nur das in den Schaufelkammern (24) vorhandene restliche Gasvolumen wird vom Laufrad (12) am Unterbrecher (14) weiter gefördert.

### Bezugszeichenliste

- 1: Seitenkanalverdichter
- 2: Gehäuse
- 3: Gehäusedeckel
- 4: Zylinderkopfschraube
- 5: Rundschnurring
- 6: Elektromotor
- 7: Zentrieransatz
- 8: Zentrierbohrung
- 9: Senkkopfschraube
- 10: Senkbohrung
- 11: Motorzapfen
- 12: Laufrad
- 13: Seitenkanal
- 14: Unterbrecher
- 15: Expansionsstrecke
- 16: Zentrieransatz
- 17: Bohrung
- 18: Gewindebohrung
- 19: Anschluss
- 20: Senkung
- 21: Gas-Einlass-Öffnung
- 22: Ringnut
- 23: Eindrehung
- 24: Schaufelkammer
- 25: Gas-Auslass-Öffnung
- 26: Schaufel
- 27: Gewindebohrung für Schraube 4
- 28: Gewindebohrung zur Befestigung des Seitenkanalverdichters 1
- 29: Schaufelkammerwand
- 30: Ringnut
- 31: Querschnitt des Seitenkanals 13
- 32: Boden

## Patentansprüche

1. Geräuscharmer Seitenkanalverdichter (1) für Geräte zur Beatmungstherapie mit
a) einem Gehäuse (2)
b) einem in dem Gehäuse (2) befindlichen ringförmigen Seitenkanal (13) mit einem Querschnitt (31) zum Verdichten eines Gases,
c) einem durch einen Elektromotor (6) drehangetriebenen, scheibenförmigen Laufrad (12) mit einer Vielzahl auf einem gemeinsamen Teilkreis liegender Schaufelkammern (24), die dem Seitenkanal (13) gegenüberliegen und auf dem Teilkreis verteilt sind, wobei der Durchmesser des Laufrades (12) ca. 50 mm beträgt,
d) wobei die Schaufelkammern (24) durch Schaufelkammerwände (29) und einen Boden (32) gebildet werden,
e) einer im Gehäuse (2) ausgebildeten Gas-Einlass-Öffnung (21), die mit dem Seitenkanal (13) zur Einführung eines zu verdichtenden Gases in Strömungsverbindung steht,
f) einer im Gehäuse (2) ausgebildeten Gas-Auslass-Öffnung (25), die mit dem Seitenkanal (13) zur Abführung des verdichteten Gases aus dem Seitenkanal (13) in Strömungsverbindung steht,
g) einem im Seitenkanal (13) zwischen Gas-Auslass-Öffnung (25) und Gas- Einlass-Öffnung (21) liegenden Unterbrecher (14),
**dadurch gekennzeichnet, dass**
h) die die einzelnen Schaufelkammern (24) trennenden Schaufelkammerwände (29) zum Laufradumfang hin derart dicker werden, dass sich deren ins Innere benachbarter Schaufelkammern (24) gerichtete Oberflächen parallel zu den radial verlaufenden Mittelebenen dieser Schaufelkammern (24) erstrecken.

2. Geräuscharmer Seitenkanalverdichter (1) nach Patentanspruch 1,
**dadurch gekennzeichnet, dass**
die Schaufelkammern (24) in radialer Richtung jeweils einen im Querschnitt einer Kurve folgenden Boden (32) aufweisen und die Übergänge zwischen den Böden (32) der einzelnen Schaufelkammern (24) und deren Schaufelkammerwänden (29), mit Kurven versehen sind.

3. Geräuscharmer Seitenkanalverdichter (1) nach einem der vorhergehenden Patentansprüche 1 oder 2,
**dadurch gekennzeichnet, dass**
das Laufrad (12) und das Gehäuse (2) jeweils mindestens einen Teilkreis aufweisen.

4. Geräuscharmer Seitenkanalverdichter (1) nach einem der vorhergehenden Patentansprüche 1 bis 3,
**dadurch gekennzeichnet, dass**
im Laufrad (12) mindestens 25, vorzugsweise 36, Schaufelkammern (24) eingebracht sind.

5. Geräuscharmer Seitenkanalverdichter (1) nach einem der vorhergehenden Patentansprüche 1 bis 4,
**dadurch gekennzeichnet, dass**
die Drehzahl n des Laufrades (12) ca. 30000 1/min beträgt.

6. Geräuscharmer Seitenkanalverdichter (1) nach einem der vorhergehenden Patentansprüche 1 bis 5,
**dadurch gekennzeichnet, dass**
die Oberflächen der gasführenden Teile, insbesondere der Seitenkanal (13) und die Schaufelkammern (24), hydraulisch glatt ausgeführt sind.

7. Geräuscharmer Seitenkanalverdichter (1) nach einem der vorhergehenden Patentansprüche 1 bis 6,
**dadurch gekennzeichnet, dass**
die Oberflächen der gasführenden Teile, insbesondere der Seitenkanal (13) und die Schaufelkammern (24), antibakteriell ausgeführt sind.

8. Geräuscharmer Seitenkanalverdichter (1) nach einem der vorhergehenden Patentansprüche 1 bis 7,
**dadurch gekennzeichnet, dass**
das Gehäuse (2) und das Laufrad (12) glatte Oberflächen besitzen, und am Ende des Unterbrechers (14) eine Expansionsstrecke (15) des verdichteten Gases vorgesehen ist.

9. Geräuscharmer Seitenkanalverdichter (1) nach einem der vorhergehenden Patentansprüche 1 bis 8,
**dadurch gekennzeichnet, dass**
an der Gas-Einlass-Öffnung (21) und/oder der Gas-Auslass-Öffnung (25) zusätzliche Schalldämpfer angeschlossen werden können.

10. Geräuscharmer Seitenkanalverdichter (1) nach einem der vorhergehenden Patentansprüche 1 bis 9,
**dadurch gekennzeichnet, dass**
Werkstoffe mit geringer Dichte und gleichzeitig hoher Festigkeit, insbesondere beim Laufrad (12), verwendet werden.

11. Gerät zur Beatmungstherapie, **dadurch gekennzeichnet, dass** ein Seitenkanalverdichter (1) nach einem der vorhergehenden Patentansprüche 1 bis 10 eingebaut ist.

## Claims

1. A low-noise side channel compressor (1) for devices for ventilation therapy, comprising
a) a housing (2);
b) an annular side channel (13) located within the housing (2) and having a cross-section (31) for compressing a gas;
c) a disk-shaped impeller (12) driven to rotate by an electric motor (6) and having a plurality of blade chambers (24) that are located on a shared pitch circle, are arranged opposite of the side channel (13) and are distributed across the pitch circle, the diameter of the impeller (12) being about 50 mm;
d) the blade chambers (24) being formed by blade chamber walls (29) and a bottom (32);
e) a gas inlet opening (21) formed in the housing (2) and being in fluid communication with the side channel (13) for introducing a gas to be compressed;
f) a gas outlet opening (25) formed in the housing (2) and being in fluid communication with the side channel (13) for discharging the compressed gas from the side channel (13);
g) an interrupter (14) located in the side channel (13) between the gas outlet opening (25) and the gas inlet opening (21);
**characterised in that**
h) the blade chamber walls (29) separating the individual blade chambers (24) become thicker toward the circumference of the impeller in such a manner that their surfaces oriented into the inside of adjacent blade chambers (24) extend parallel to the radially running centre planes of said blade chambers (24).

2. The low-noise side channel compressor (1) according to claim 1,
**characterised in that**
in the radial direction, each of the blade chambers (24) has a bottom (32) that follows a curve in the cross-section, and the transitions between the bottoms (32) of the individual blade chambers (24) and their blade chamber walls (29) are provided with curves.

3. The low-noise side channel compressor (1) according to any one of the preceding claims 1 or 2,
**characterised in that**
the impeller (12) and the housing (2) each have at least one pitch circle.

4. The low-noise side channel compressor (1) according to any one of the preceding claims 1 to 3,
**characterised in that**
at least 25, preferably 36 blade chambers (24) are realised in the impeller (12).

5. The low-noise side channel compressor (1) according to any one of the preceding claims 1 to 4,
**characterised in that**
the speed of rotation n of the impeller (12) is about 30,000 rpm.

6. The low-noise side channel compressor (1) according to any one of the preceding claims 1 to 5,
**characterised in that**
the surfaces of the gas-conducting parts, in particular the side channel (13) and the blade chambers (24), are designed to be hydraulically smooth.

7. The low-noise side channel compressor (1) according to any one of the preceding claims 1 to 6,
**characterised in that**
the surfaces of the gas-conducting parts, in particular the side channel (13) and the blade chambers (24), are designed to be antibacterial.

8. The low-noise side channel compressor (1) according to any one of the preceding claims 1 to 7,
**characterised in that**
the housing (2) and the impeller (12) have smooth surfaces, and an expansion section (15) of the compressed gas is provided at the end of the interrupter (14).

9. The low-noise side channel compressor (1) according to any one of the preceding claims 1 to 8,
**characterised in that**
additional sound absorbers can be connected at the gas inlet opening (21) and/or at the gas outlet opening (25).

10. The low-noise side channel compressor (1) according to any one of the preceding claims 1 to 9,
**characterised in that**
materials of low density and at the same time of high strength are used in particular in the impeller (12).

11. A device for ventilation therapy,
**characterised in that**
a side channel compressor (1) according to any one of the preceding claims 1 to 10 is built in.

## Revendications

1. Compresseur à canal latéral (1) peu bruyant pour des dispositifs de thérapie ventilatoire, comprenant
a) un boîtier (2) ;
b) un canal latéral (13) annulaire situé dans le boîtier (2) et ayant une section transversale (31) pour comprimer un gaz ;
c) une turbine (12) sous forme de disque, entraînée en rotation par un moteur électrique (6) et ayant une pluralité de chambres d'aube (24) qui sont situées sur un cercle primitif commun, qui font face au canal latéral (13) et qui sont distribuées sur le cercle primitif, le diamètre de la turbine (12) étant d'environ 50 mm ;
d) les chambres d'aube (24) étant formées par des parois de chambre d'aube (29) et un fond (32) ;
e) une ouverture d'entrée de gaz (21) formée dans le boîtier (2) et étant en communication fluide avec le canal latéral (13) pour l'introduction d'un gaz à comprimer ;
f) une ouverture de sortie de gaz (25) formée dans le boîtier (2) et étant en communication fluide avec le canal latéral (13) pour l'évacuation du gaz comprimé du canal latéral (13) ;
g) un interrupteur (14) situé dans le canal latéral (13) entre l'ouverture de sortie de gaz (25) et l'ouverture d'entrée de gaz (21) ;
**caractérisé en ce que**
h) les parois de chambre d'aube (29) séparant les chambres d'aube (24) individuelles grossissent vers la circonférence de la turbine de telle manière que leurs surfaces tournées vers l'intérieur des chambres d'aube (24) adjacentes s'étendent parallèlement aux plans médians desdites chambres d'aube (24), lesdits plans médians s'étendant radialement.

2. Compresseur à canal latéral (1) peu bruyant selon la revendication 1,
**caractérisé en ce que**
dans la direction radiale, chaque chambre d'aube (24) a un fond (32) qui suit une courbe en section transversale, et les zones de transition entre les fonds (32) des chambres d'aube (24) individuelles et leurs parois de chambre d'aube (29) présentent des courbes.

3. Compresseur à canal latéral (1) peu bruyant selon l'une quelconque des revendications précédentes 1 ou 2,
**caractérisé en ce que**
la turbine (12) et le boîtier (2) ont chacun au moins un cercle primitif.

4. Compresseur à canal latéral (1) peu bruyant selon l'une quelconque des revendications précédentes 1 à 3,
**caractérisé en ce**
**qu'**au moins 25, de préférence 36 chambres d'aube (24) sont réalisées dans la turbine (12).

5. Compresseur à canal latéral (1) peu bruyant selon l'une quelconque des revendications précédentes 1 à 4,
**caractérisé en ce que**
la vitesse de rotation n de la turbine (12) est d'environ 30.000 tr/min.

6. Compresseur à canal latéral (1) peu bruyant selon l'une quelconque des revendications précédentes 1 à 5,
**caractérisé en ce que**
les surfaces des pièces conduisant le gaz, notamment le canal latéral (13) et les chambres d'aube (24), sont hydrauliquement lisses.

7. Compresseur à canal latéral (1) peu bruyant selon l'une quelconque des revendications précédentes 1 à 6,
**caractérisé en ce que**
les surfaces des pièces conduisant le gaz, notamment le canal latéral (13) et les chambres d'aube (24), sont antibactériennes.

8. Compresseur à canal latéral (1) peu bruyant selon l'une quelconque des revendications précédentes 1 à 7,
**caractérisé en ce que**
le boîtier (2) et la turbine (12) ont des surfaces lisses, et une section d'expansion (15) du gaz comprimé est disposée à l'extrémité de l'interrupteur (14).

9. Compresseur à canal latéral (1) peu bruyant selon l'une quelconque des revendications précédentes 1 à 8,
**caractérisé en ce que**
des amortisseurs de bruit additionnels peuvent être connectés à l'ouverture d'entrée de gaz (21) et/ou à l'ouverture de sortie de gaz (25).

10. Compresseur à canal latéral (1) peu bruyant selon l'une quelconque des revendications précédentes 1 à 9,
**caractérisé en ce que**
des matériaux de faible densité et, simultanément, à haute résistance sont utilisés, notamment dans la turbine (12).

11. Dispositif de thérapie ventilatoire,
**caractérisé en ce**
**qu'**un compresseur à canal latéral (1) selon l'une quelconque des revendications précédentes 1 à 10 est incorporé.
